# EUROPEAN PATENT APPLICATION

(11) **EP 3 318 256 A1**
(43) Date of publication of application: **09.05.2018**
(21) Application number: 16382518.5
(22) Date of filing: 08.11.2016
(51) Int. Cl.: A61K 31/401, A61K 31/402, A61K 31/4025, A61K 31/4439, A61P 37/00, A61P 19/02, A61P 3/10, A61P 25/00

(54) **COMPOUNDS FOR THE TREATMENT OF AUTOIMMUNE DISEASES**

(71) Applicant: QUIMATRYX, S.L, 20009 San Sebastián (ES)
(72) Inventor: VARA SALAZAR, Yosu Ion, E-20009 San Sebastian, Guipuzcoa (ES); ALDABA ARÉVALO, Eneko, E-20009 San Sebastian, Guipuzcoa (ES); NICOLETTI, Ferdinando, 95021 Cannizzaro-Acicastello (IT); LIBRA, Massimo, 95123 Catania (IT); MANGANO, Katia Domenica, 95128 Catania (IT)
(74) Representative: ABG Patentes, S.L.

(57) **Abstract**

The present invention refers to the use of compounds derived from trisubstituted 1*H*-pyrrole rings and aromatic rings, which have the following formula (I): 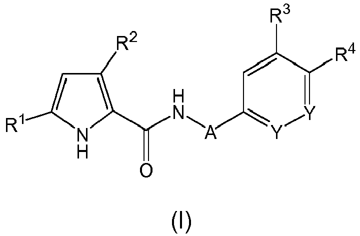 wherein
R¹ and R² represent, independently of each other:
an optionally substituted phenyl ring, or
an optionally substituted 5-membered heteroaromatic ring;

one of R³ and R⁴ is H and the other represents a 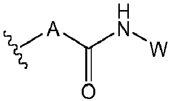 group wherein W is OH or an *ortho*-aniline group;
each A, independently of each other, represents a -CH₂- group or is absent; =Y-Y= is =HC-CH=, =N-CH=, or =HC-N=;
or a salt or solvate thereof,
in the prevention or treatment of an autoimmune disease.

## Description

### Field of the invention

The present invention relates to compounds derived from trisubstituted pyrroles and conformationally restricted intermediate aromatic rings, and to the use of said compounds for the prevention or treatment of autoimmune diseases.

### Background of the invention

Autoimmune diseases form a group of diseases caused by an impairment of the immune system that makes it react against the body's own tissues.

The modification of the cell recognition mechanisms that normally enable the body to distinguish between the "self" and the "non-self", i.e. between elements that belong to the body and elements that are foreign to it, give rise to the production of antibodies, which can target single organs (organ-specific diseases) or trigger systemic disorders, damaging the individual's functions as a whole.

Treatment of autoimmune diseases depends on the type and severity of the condition. Nonsteroidal anti-inflammatory drugs (NSAIDs), steroids and immunosuppressants are often used. Intravenous Immunoglobulin may also occasionally be used. For instance, in the case of multiple sclerosis, Interferon (IFN)-beta, Glatiramer acetate, Cyclophosphamide or Mitoxantrone are typically employed. Attempts at providing new treatments against autoimmune diseases include the use of proteins or small organic compounds as immunomodulators. For instance, WO 2011/098990 discloses the use of apotransferrin for treating autoimmune diseases.

However, while reported treatments usually improve symptoms they do not typically cure the disease, and in some cases side-effects of the treatment can be just as deleterious as the disease itself. In particular, genotoxic side-effects, such as clastogenicity or aneugenicity, which are typically associated to treatments that interfere with epigenetic regulation, are of particular importance due to their seriousness and potential lethality.

There is a constant and urgent need to develop new treatments against autoimmune diseases which enrich the plethora of existing therapies and preferably overcome drawbacks associated to existing therapies.

### Summary of the invention

The present inventors have identified a series of small organic compounds which have unexpectedly been proven useful in the prevention or treatment of different autoimmune diseases.

Thus, in an aspect, the present invention relates to compounds of general formula (I): 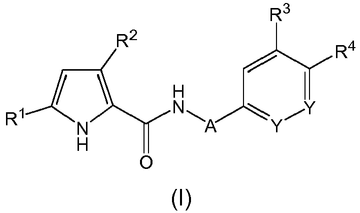 wherein
R¹ and R² represent, independently of each other:
   an optionally substituted phenyl ring, or
   an optionally substituted 5-membered heteroaromatic ring; one of R³ and R⁴ is H and the other represents a 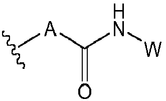 group wherein
W is OH or an *ortho*-aniline group;
each A, independently of each other, represents a -CH₂- group or is absent (A could alternatively be represented as (CH₂)₀₋₁;
=Y-Y= is =HC-CH=, =N-CH=, or =HC-N=;
or a salt or solvate thereof,
for use in the prevention or treatment of an autoimmune disease.

Another aspect of the present invention relates to the use of a compound of general formula (I), or a salt or solvate thereof, in the preparation of a medicament for the prevention or treatment of an autoimmune disease.

According to another aspect, the present invention relates to a method of preventing or treating an autoimmune disease, the method comprising administering to a patient in need of such treatment a therapeutically effective amount a compound of general formula (I) or a salt or solvate thereof.

### Brief description of the drawings

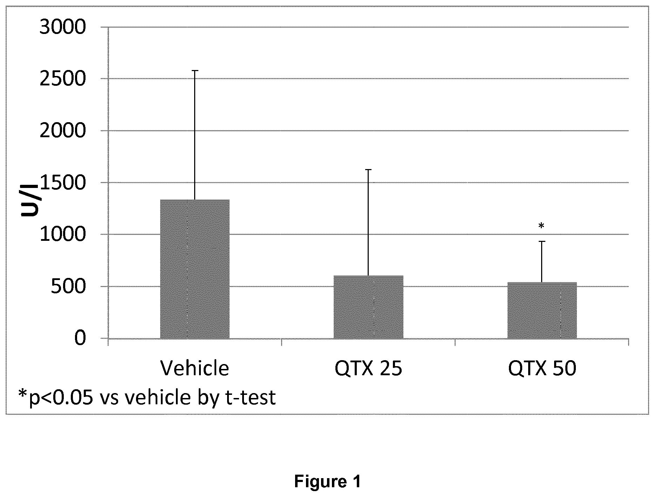

Figure 1. In vivo efficacy results for a compound according to the invention in a first animal model (Example 1 of the present application). Y-axis: light emission in units/L. X-axis (tested compositions): vehicle (no compound according to the invention); QTX 25 - compound according to the invention (QTX125) at 25 mg/kg; QTX 50 - compound according to the invention (QTX125) at 50 mg/kg.

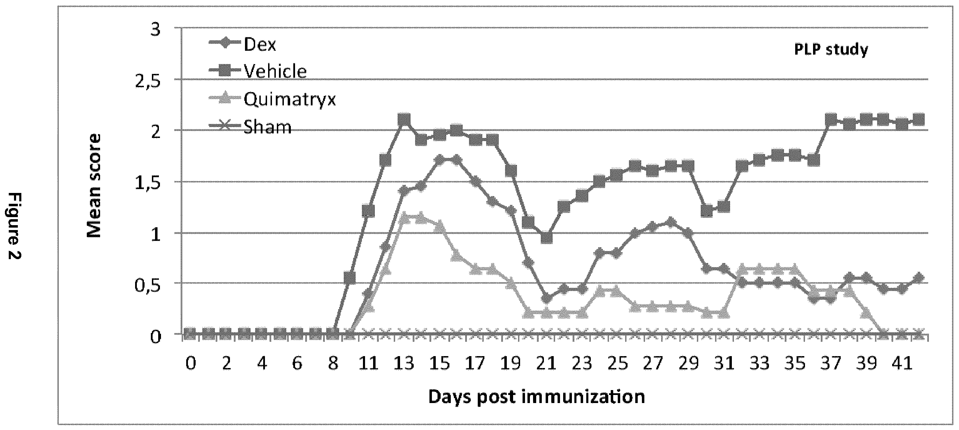

Figure 2. In vivo efficacy results for a compound according to the invention in a second animal model (Example 2 of the present application). Y-axis: EAE clinical grading; X-axis: days post-immunization. Dex: Dexamethasone; Vehicle: no compound according to the invention; Quimatryx: compound according to the invention (QTX125); Sham: negative control.

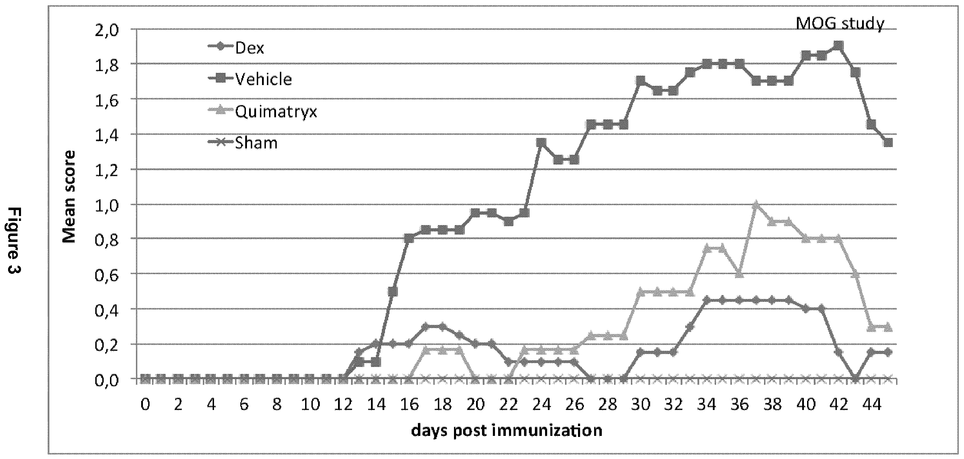

Figure 3. In vivo efficacy results for a compound according to the invention in a third animal model (Example 3 of the present application). Y-axis: EAE clinical grading; X-axis: days post-immunization. Dex: Dexamethasone; Vehicle: no compound according to the invention; Quimatryx: compound according to the invention (QTX125); Sham: negative control.

### Detailed description of the invention

In a preferred embodiment the compound of general formula (I) is a compound of general formula (II): 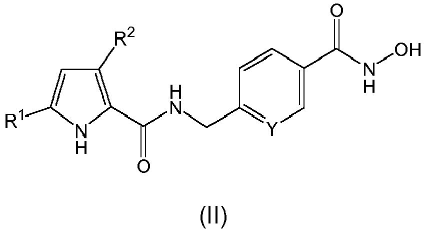 wherein
R¹ and R² are as defined for formula (I);
=Y- is =CH- or =N-, preferably =CH-;
or a salt or solvate thereof.

In any of the above embodiments, the optionally substituted phenyl ring that R¹ or R² can represent is preferably:
- a non-substituted phenyl ring; or
- a phenyl ring substituted with:
   ∘ C₁-C₆ alkyl group; halogen; nitro; cyano; trifluoromethyl; OR', SR', SOR', SO₂R', NR'R", C(O)R', C(O)OR', C(O)NR'R" or OC(O)R', where R' and R" are selected, independently, from a hydrogen atom and a C₁-C₆ alkyl group, preferably methyl;
      or more preferably
   ∘ OH; OR⁵, wherein R⁵ is a C₁-C₆ alkyl group, preferably methyl; or halogen, preferably F;
      or even more preferably
   ∘ OH.
   In a preferred embodiment, substitution is monosubstitution. In a preferred embodiment substitution is para-substitution. In particular, it can be *para-*monosubstitution Even more preferably, substitution is *para*-OH monosubstitution. In a preferred embodiment, R¹ is an optionally substituted phenyl ring as described above.

In any of the above embodiments, the optionally substituted 5-membered heteroaromatic ring that R¹ or R² can represent is a furan ring, a thiophene ring or a pyrrole ring, preferably a furan ring or a thiophene ring, most preferably a furan ring. If substituted, substituents are chosen as from the following list:
∘ C₁-C₆ alkyl group; halogen; nitro; cyano; trifluoromethyl; OR', SR', SOR', SO₂R', NR'R", C(O)R', C(O)OR', C(O)NR'R" or OC(O)R', where R' and R" are selected, independently, from a hydrogen atom and a C₁-C₆ alkyl group, preferably methyl;
   or more preferably
∘ OH; OR⁵, wherein R⁵ is a C₁-C₆ alkyl group, preferably methyl; or halogen, preferably F;
   or even more preferably
∘ OH.

Preferably, the heteroaromatic ring is not substituted. Preferably, the furan, thiophene or pyrrole ring is bound to the rest of the molecule at position 2 or 3 of said furan, thiophene or pyrrole, even more preferably at position 3 (numbering shown below). Most preferably, the heteroaromatic ring is a furan ring bound to the rest of the molecule at position 3 of said furan ring. In a preferred embodiment, R² is an optionally substituted 5-membered heteroaromatic ring as described above.

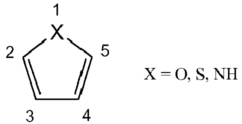

In a most preferred embodiment, R¹ is a *para*-OH-monosubstituted phenyl ring, and R² is a furan ring bound to the rest of the molecule at position 3 of said furan ring.

In specific embodiments, the compound of formula (I) is selected from the following list: 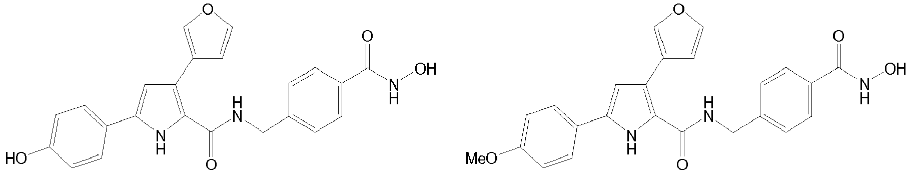 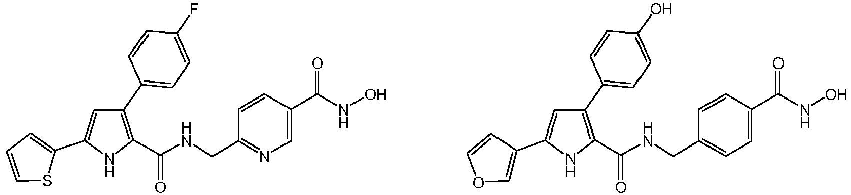 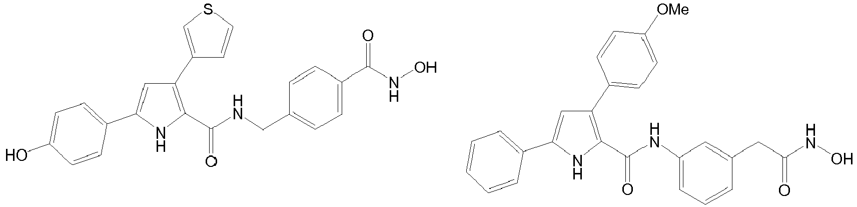 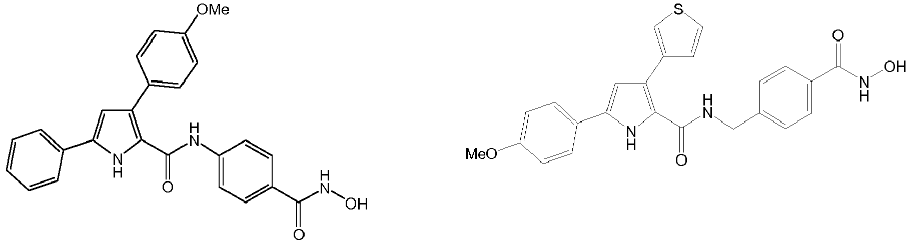 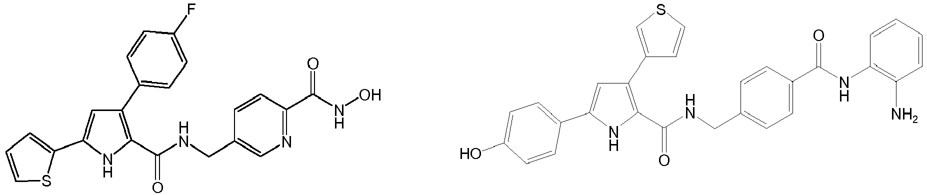 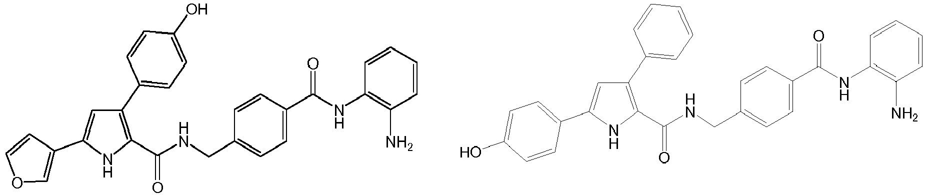 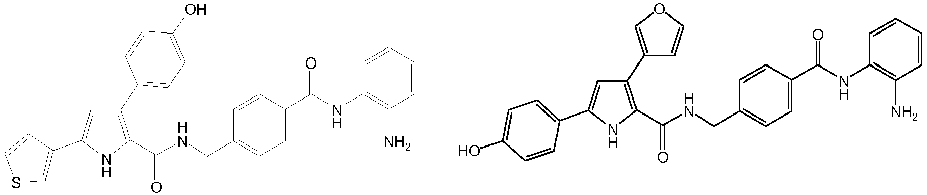 or a salt or solvate thereof.

In preferred specific embodiments, the compound of formula (I) is selected from the following list: 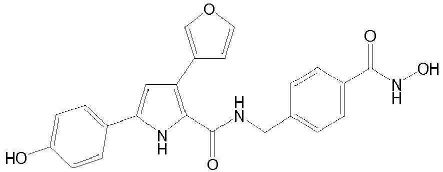 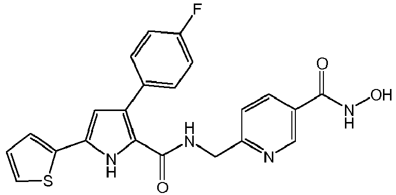 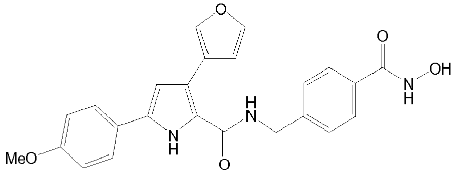 or a salt or solvate thereof.

More preferably, the compound of formula (I) is 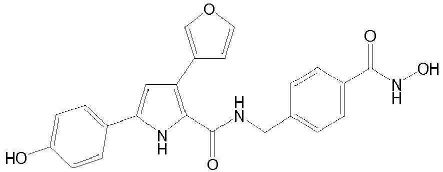 (hereinafter referred to as QTX125) or a salt or solvate thereof. Most preferably, the compound of formula (I) is QTX125.

In the context of the present invention, the following terms have the meanings detailed below:
The term "C₁-C₆ alkyl" refers to a linear or branched hydrocarbon chain consisting of carbon and hydrogen atoms, containing no unsaturation, having from one to six carbon atoms, and which is attached to the rest of the molecule through a single bond, including for example and in a non limiting sense, methyl, ethyl, n-propyl, i-propyl, n-butyl, t-butyl, or n-pentyl.

"Heteroaromatic ring" refers to a stable aromatic ring which consists of carbon atoms and from one to four heteroatoms selected from nitrogen, oxygen and sulfur.

The term "halogen" refers to bromine (Br), chlorine (Cl), iodine (I) or fluorine (F).

An "*ortho*-aniline" group refers to the following moiety:

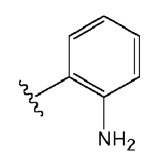

The compounds of formula (I) may be in the form of salts, preferably as pharmaceutically acceptable salts, or as solvates.

The term "pharmaceutically acceptable salts" refers to salts which, when administered to the recipient, can provide (directly or indirectly) a compound as described in the present document. "Pharmaceutically acceptable" preferably refers to compositions and molecular entities that are physiologically tolerable and do not usually produce an allergic reaction or a similar unfavorable reaction as gastric disorders, dizziness and suchlike, when administered to a human or animal. Preferably, the term "pharmaceutically acceptable" means it is approved by a regulatory agency of a state or federal government or is included in the U.S. Pharmacopoeia or other generally recognized pharmacopoeia for use in animals, and more particularly in humans.

The preparation of salts can be accomplished by methods known in the art. For example, the pharmaceutically acceptable salts of compounds provided herein are synthesized from the original compound, which contains basic residues, by conventional chemical methods. Generally, such salts are prepared, for example, by reacting free base forms of these compounds with the appropriate base or acid in water or in an organic solvent or in a mixture of both. In general, non-aqueous media like ether, ethyl acetate, ethanol, isopropanol or acetonitrile are preferred. Examples of acid addition salts include mineral acid addition salts such as, e.g. hydrochloride, hydrobromide, hydroiodide, sulfate, nitrate, phosphate salts and organic acid addition salts such as, for example, acetate, maleate, fumarate, citrate, oxalate, succinate, tartrate, malate, mandelate, methanesulfonate and p-toluenesulfonate salts. Examples of base addition salts include inorganic salts such as, for example, sodium, potassium, calcium, ammonium, magnesium, aluminum and lithium salts, and organic salts such as, for example, ethylenediamine, ethanolamine, *N*,*N*-dialkylenethanolamine, triethanolamine, glucamine and basic salts of aminoacids.

The term "solvate" according to this invention is to be understood as any form of the active compound of the invention which has another molecule (most likely a polar solvent) attached to it through noncovalent bonds. Examples of solvates include hydrates and alcoholates, for example methanolate. The compounds of the invention can be in crystalline form, either as free compounds or as solvates. Methods of solvation are known within the art. In a particular embodiment the solvate is a hydrate.

The compounds of the invention can be prepared as described in WO 2011/039353 A1, the teachings of which are herein incorporated by reference.

According to the present invention, the compounds of formula (I) (or salts or solvates thereof) are used in the prevention or treatment of an autoimmune disease.

In an embodiment, the autoimmune disease is selected from autoimmune hepatitis; an inflammatory demyelinating disease of the central nervous system; systemic lupus erythematosus; acute anterior uveitis; Sjögren's syndrome; rheumatoid arthritis; diabetes mellitus type 1; Graves' disease; and inflammatory bowel disease.

An inflammatory demyelinating disease of the central nervous system is a disease wherein myelin-supporting cells of the central nervous system, such as oligodendrocytes, and/or the myelin lamellae are destroyed. Demyelination leads to a disruption in neural signals between the brain and other parts of the body, ultimately resulting in a range of signs and symptoms, including physical, mental, and sometimes psychiatric problems.

Specific, non-limiting examples of inflammatory demyelinating diseases are multiple sclerosis (MS), including relapsing-onset MS, progressive-onset MS, optic-spinal MS; neuromyelitis optica; acute-disseminated encephalomyelitis; acute hemorrhagic leukoencephalitis; Balo concentric sclerosis; Schilder's disease; Marburg MS; tumefactive MS; solitary sclerosis; optic neuritis; transverse myelitis; Susac's syndrome; leukoaraiosis; myalgic encephalomyelitis; Guillain-Barré syndrome; progressive inflammatory neuropathy; leukodystrophy, including adrenoleukodystrophy and adrenomyeloneuropathy. Preferably, the autoimmune disease is multiple sclerosis or acute-disseminated encephalomyelitis. More particularly it is acute-disseminated encephalomyelitis, or more particularly and most preferably it is multiple sclerosis.

In a preferred embodiment, the autoimmune disease is selected from autoimmune hepatitis and an inflammatory demyelinating disease of the central nervous system.

In a particularly preferred embodiment, the autoimmune disease is an inflammatory demyelinating disease of the central nervous system as described above.

In another particularly preferred embodiment, the autoimmune disease is autoimmune hepatitis.

The present inventors have found that the compounds used in the present invention, unlike other histone deacetylase inhibitors, advantageously show no evidence of genotoxicity, in particular of clastogenicity or aneugenicity. Similarly, it has unexpectedly been observed that the compounds used in the present invention possess improved pharmacokinetic properties, in particular higher half-lives and distribution volumes, than other histone deacetylase inhibitors.

The term "treatment" or "treating" in the context of this document means administration of a compound or a pharmaceutical composition of the invention to improve or eliminate the disease or one or more symptoms associated with the disease. The term "prevention" or "prevent" includes reducing the risk of the disease appearing or developing.

The compounds of the present invention are administered in the form of a pharmaceutical composition. A pharmaceutical composition may comprise one or more compounds according to the present invention. Thus, the invention also relates to a pharmaceutical composition comprising at least one compound of general formula (I) or a salt or solvate thereof, and at least one pharmaceutically acceptable excipient, for use in the treatment or prevention of an autoimmune disease as previously described.

The term "excipient" refers to a vehicle, diluent or adjuvant that is administered with the active ingredient. Such pharmaceutical excipients can be sterile liquids, such as water and oils, including those of petroleum, animal, vegetable or synthetic origin, such as peanut oil, soybean oil, mineral oil, sesame oil or similar. Water or saline aqueous solutions and aqueous dextrose and glycerol, particularly for injectable solutions, are preferably used as vehicles. Suitable pharmaceutical vehicles are described in "Remington's Pharmaceutical Sciences" by E.W. Martin, 1995.

Examples of pharmaceutical compositions include any solid composition (tablets, pills, capsules, granules, etc.) or liquid composition (solutions, suspensions or emulsions). Routes of administration useful for the treatment of the present invention include but are not limited to oral and parenteral routes, such as intravenous (iv), intraperitoneal (ip), rectal, topical, ophthalmic, nasal, and transdermal.

In a preferred embodiment the pharmaceutical compositions are in oral delivery form or are administered orally. Pharmaceutical forms suitable for oral administration may be tablets and capsules and may contain conventional excipients known in the art such as binders, for example syrup, gum arabic, gelatin, sorbitol, tragacanth or polyvinylpyrrolidone; fillers, for example lactose, sugar, cornstarch, calcium phosphate, sorbitol or glycine; lubricants for the preparation of tablets, for example magnesium stearate; disintegrants, for example starch, polyvinylpyrrolidone, sodium starch glycolate or microcrystalline cellulose; or pharmaceutically acceptable wetting agents such as sodium lauryl sulphate.

Solid oral compositions can be prepared by conventional methods of blending, filling or preparation of tablets. Repeated blending operations can be used to distribute the active ingredient in all the compositions that use large amounts of fillers. Such operations are conventional in the art. The tablets can be prepared, for example, by dry or wet granulation and optionally can be coated by well known methods in normal pharmaceutical practice, in particular using a enteric coating.

Pharmaceutical compositions can also be adapted for parenteral administration, such as sterile solutions, suspensions or lyophilized products in the appropriate unit dosage form. Suitable excipients such as fillers, buffering agents or surfactants can be used.

In a preferred embodiment, compositions are in parenteral delivery form or are administered parenterally. Preferably, compositions are in intravenous delivery form or are administered intravenously. Intravenous delivery forms include, but are not limited to, bolus and drip injections. Examples of intravenous dosage forms include, but are not limited to, water, preferably sterile water for injection USP; aqueous vehicles including, but not limited to, sodium chloride injection, Ringer's injection, dextrose injection, dextrose and sodium chloride injection, and lactated Ringer's injection; water-miscible vehicles including, but not limited to, ethyl alcohol, polyethylene glycol and polypropylene glycol; and non-aqueous vehicles including, but not limited to, corn oil, cottonseed oil, peanut oil, sesame oil, ethyl oleate, isopropyl myristate and benzyl benzoate. In preferred embodiments, the intravenous dosage forms are sterile or capable of being sterilized prior to administration to a subject since they typically bypass the subject's natural defenses against contaminants.

The mentioned formulations will be prepared using standard methods such as those described or referred to in the Spanish and U.S. Pharmacopoeias and similar reference texts.

In general, the effective amount of a compound of the invention to be administered will depend on the relative efficacy of the compound chosen, the nature and severity of the disorder being treated and the subject's weight, or the mode of administration. However, the active compounds will normally be administered one or more times a day for example 1, 2, 3 or 4 times daily, with typical total daily doses in the range from 0.01 up to 1,000 mg/kg/day. In a preferred embodiment, the daily doses range from 1 to 100 mg/kg/day, more preferably 70 to 10 mg/kg/day. In a particular embodiment, the daily doses range from 30 to 20 mg/kg/day. In a different particular embodiment, the daily doses range from 70 to 50 mg/kg/day.

The compounds of the present invention can be formulated for use in human or veterinary medicine.

The compounds of the present invention can be used with at least another treatment or drug known to be useful against an autoimmune disease to provide a combination therapy. The other treatment or drug can be administered at the same time or at a time different to the time of administration of the compound according to the present invention. If another drug is used, the compound of the invention and the other drug may be part of the same composition, or may be provided as separate compositions.

In order to facilitate the understanding of the preceding ideas, some examples of embodiments of the present invention are described below. These examples are merely illustrative.

### Examples

### Example 1: Effect of compound of formula (I) in an animal model of autoimmune hepatitis

Concanavalin A (ConA)-induced hepatitis is a cell-mediated immunoinflammatory condition that can be induced in mice by a single intravenous injection of ConA. It is typically employed as a model for autoimmune hepatitis. The disease is characterized by a marked increase in the plasma levels of transaminase (GPT) shortly (8-24 hours) after ConA challenge and simultaneous infiltration of the liver with neutrophils, macrophages and T cells followed by apoptosis and necrosis of the hepatocytes. The aim of the present example was to evaluate the effects of a compound of formula (I) (QTX125) on histological and clinical parameters in the model of autoimmune hepatitis induced by ConA.

Male Balb/C mice 7-8 weeks old (Envigo, San Pietro al Natisone, Udine, Italy) were kept under standard laboratory conditions (non-specific pathogen free) with free access to food and water and were allowed to adapt at least one week to their environment before commencing the study.

Animals were housed within a limited access rodent facility under controlled microbial conditions which excluded murine pathogens and kept in groups of maximum 5 mice, in polycarbonate cages (Tecniplast, Varese, Italy) according to Italian legislation. This regime does not exclude various species of helicobacter. Cages were sterilized and filled with wood shavings as bedding material. Automatically controlled environmental conditions were set to maintain temperature at 20-24°C with a relative humidity (RH) of 30-70%, 10-30 air changes /hr and a natural dark:light cycle.

Protection of animals used in the experiment was in accordance with Directive 2010/63/UE. Physical facilities and equipment for accommodation and care of animals were in accordance with the provisions of EEC Council Directive 86/609.

ConA (Sigma Chemical, St. Louis, MO), was dissolved in sterile phosphate-buffered saline (PBS) and was injected into the tail veins of the animals at the dose of 20 mg/Kg.

Three groups of 10 mice were treated at -24 and -1 hours before ConA injection as described in the table below:

**Table 1. Treatment groups corresponding to each composition tested.**

| **Group** | **Number** | **Treatment** | **Dose** | **Route** | **Administration frequency** |
|---|---|---|---|---|---|
| 1 | 10 | QTX125 | 25 mg/Kg | i.p. | 24 and 1 hour before ConA |
| 2 | 10 | QTX125 | 50 mg/Kg | i.p. | 24 and 1 hour before ConA |
| 3 | 10 | Vehicle | ---- | i.p. | 24 and 1 hour before ConA |

Eight hours after ConA injection mice were sacrificed by CO₂ inhalation for blood collection for the detection of transaminases (specifically GPT) by a standard photometric assay using a bichromatic analyzer. Whole blood is added in the strip for GPT parameter. The strip is inserted into the instrument that, in approximately 2-3 minutes, measures reflectance, i.e. the intensity of the light directed onto the reagent strip and reflected all according to its depth of colour. The depth of colour is itself determined by the concentration of analyte in the sample.

Results are shown in Figure 1. As expected, treatment with vehicle (no QTX125) resulted in elevated transaminase plasma levels, as deduced from the high amount of light emission, specifically 1338 U/L. However, it was surprisingly found that mice to which QTX125 was administered produced significantly lower levels of transaminase, indicating that the prejudicial immune response to ConA injection was effectively dampened. In particular, QTX125 administration at 25 mg/Kg provided a substantial reduction of light emission down to 606 U/L, whereas administration at 50 mg/Kg resulted in an even further reduction to 543 U/L.

### Example 2: Effect of compound of formula (I) in an experimental autoimmune encephalomyelitis animal model

Experimental autoimmune encephalomyelitis (EAE) is an immunoinflammatory and demyelinating disease of the central nervous system (CNS) that it is widely used as a preclinical model of multiple sclerosis (MS), acute-disseminated encephalomyelitis (ADEM) and other inflammatory demyelinating diseases.

EAE can be induced in genetically susceptible mice by the injection of whole spinal cord, or myelin proteins such as myelin basic protein (MBP) or proteolipid protein (PLP) in complete Freund's adjuvant (CFA). The antigen-specific effector cells involved in the CNS damage are class II major histocompatibility complex (MHO) restricted CD4⁺ T lymphocytes. Upon activation by antigen, T cells produce several cytokines such as IL-2, IFN-γ and TNF-α that may be directly or indirectly responsible for the CNS damage. IL-2 has an important role in T cell activation and proliferation, while IFN-γ is a potent mediator of macrophage activation. In addition, IFN-γ induces the production of inflammatory cytokines such as IL-1, TNF, and also the expression of class II MHC molecules, among others, on the endothelial cells of blood vessels in the CNS, and on astrocytes, which are thought to play an important role in antigen presentation to encephalitogenic T cells.

From the clinical point of view, EAE in SJL mice is characterized by an acute onset that usually progresses from flaccid tail (score 1) to total hind limb paralysis and moderate forelimb weakness (score 4), or even quadriplegia and/or death (score 5). However, historical data show that the mean maximal clinical score rarely exceeds a score of 2.5 and forelimb paresis is seen in <10% of animals. From the histopathological point of view, SJL-EAE is characterized by perivascular inflammatory infiltrates and demyelination in the central nervous system (CNS).

The aim of the present example was to evaluate the effects of QTX125 compound on the clinical course of PLP-induced EAE.

Female SJL mice (Envigo, San Pietro al Natisone, Udine, Italy) were kept under standard laboratory conditions (non-specific pathogen free) with free access to food and water and were allowed to adapt at least one week to their environment before commencing the study.

Animals were housed within a limited access rodent facility under controlled microbial conditions which excluded murine pathogens and kept in groups of maximum 5 mice, in polycarbonate cages (Tecniplast, Varese, Italy) according to the Italian legislation. This regime does not exclude various species of helicobacter. Cages were sterilized and filled with wood shavings as bedding material. Automatically controlled environmental conditions were set to maintain temperature at 20-24°C with a relative humidity (RH) of 30-70%,10-30 air changes /hr and a natural dark:light cycle.

Protection of animals used in the experiment was in accordance with Directive 2010/63/UE. Physical facilities and equipment for accommodation and care of animals were in accordance with the provisions of EEC Council Directive 86/609.

Mice were treated under a late prophylactic regime from day 7 post immunization until day 42 post immunization as described in the table below:

**Table 2. Treatment groups corresponding to each composition tested.**

| **Mouse Group** | **Number of mice** | **Treatment** | **Dose** | **Route** | **Administration frequency** |
|---|---|---|---|---|---|
| 1 | 10 | Dexametha sone | 0,3 mg/Kg | i.p. | Daily for 35 consecutive days |
| 2 | 10 | Vehicle | ---- | i.p. | Daily for 35 consecutive days |
| 3 | 7 | QTX125 | 60 mg/Kg | i.p. | Daily for 35 consecutive days |
| 4 | 4 | Sham | ---- | | |

Test compounds were prepared immediately before treatment and were administered i.p. daily.

Dexamethasone (Soldesam, acquired from a local pharmacy): the stock solution 4 mg/ml was diluted in water for injection at the required concentration.

Vehicle: 10% DMSO, 35% PEG 400 and 55% water for injection.

SHAM group didn't receive treatment. The group represents a reference healthy group of mice.

PLP was obtained from Genemed synthesis, San Francisco CA. EAE was induced as described by J. St. Louis et al. (J. Neuroimmunol., 2001, 115(1-2):79-90). The mice were immunized with 75 µg PLP emulsified in CFA with 6 mg/mL *Mycobacterium tuberculosis* H37RA (Difco, Detroit, MI, USA) to make a 1:1 emulsion. Except in the Sham group, each mouse received subcutaneous injections of 200 µL emulsion divided among four sites draining into the axillary and inguinal lymph nodes. Pertussis toxin (Calbiochem, Nottingham, UK) was used as a co-adjuvant and was administered i.p. at the dose of 200 ng/mouse on day 0 and 2 post immunization.

The mice were observed by measuring their body weights and clinical signs of EAE until 42 days after immunization. This EAE clinical grading was carried out by an observer unaware of the treatment and according to the following scale: 0 = no illness, 1 = flaccid tail, 2 = moderate paraparesis, 3 = severe paraparesis, 4 = forelimb paresis or moribund state, 5 = death.

The results are shown in Figure 2. As was expected, mice that underwent no PLP-immunization protocol nor received any treatment remained asymptomatic throughout the study (SHAM entry). Animals that underwent PLP-immunization and were only treated with vehicle became paraparetic. However, treatment with anti-inflammatory immunosupressant dexamethasone resulted in considerable improvement of mouse condition. Unexpectedly, treatment with a compound of formula (I) (QTX125) also resulted in substantial improvement of mouse condition with respect to mice treated with vehicle, and more surprisingly mouse condition was even ameliorated with respect to dexamethasone for the greater part of the treatment, and particularly at the end of the treatment. These results demonstrate the usefulness of the compounds of the present invention in the treatment of inflammatory demyelinating diseases of the central nervous system such as multiple sclerosis.

### Example 3: Effect of compound of formula (I) in an experimental autoimmune encephalomyelitis animal model

A variant of the animal model of Example 2 was tested. In particular, experimental autoimmune encephalomyelitis was induced by subcutaneous injection of myelin oligodendrocyte glycoprotein (MOG) instead of PLP. As in example 2, administration to mice of a compound of formula (I) (QTX125) lead to reduced paralysis scores, thus confirming the usefulness of the compounds of the present invention in the treatment of inflammatory demyelinating diseases of the central nervous system such as multiple sclerosis. Results are shown in Figure 3.

## Claims

1. A compound of general formula (I) 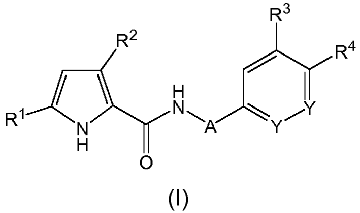 wherein
R¹ and R² represent, independently of each other:
an optionally substituted phenyl ring, or
an optionally substituted 5-membered heteroaromatic ring; one of R³ and R⁴ is H and the other represents a 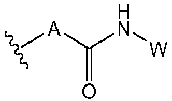 group wherein W is OH or an *ortho*-aniline group;
each A, independently of each other, represents a -CH₂- group or is absent; =Y-Y= is =HC-CH=, =N-CH=, or =HC-N=;
or a salt or solvate thereof,
for use in the prevention or treatment of an autoimmune disease.

2. A compound for use according to claim 1, which is a compound of general formula (II): 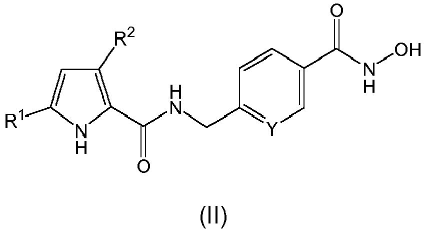 wherein
R¹ and R² are as defined in claim 1; =Y- is =CH- or =N-;
or a salt or solvate thereof.

3. A compound for use according to claim 1 or 2, wherein the optionally substituted phenyl ring is a non-substituted phenyl ring or a phenyl ring substituted with OH; OR⁵, wherein R₅ is a C₁-C₆ alkyl group; or halogen.

4. A compound for use according to any preceding claim, wherein the optionally substituted 5-membered heteroaromatic ring is a non-substituted furan, a non-substituted thiophene or a non-substituted pyrrole ring.

5. A compound for use according to claim 1, selected from the following list: 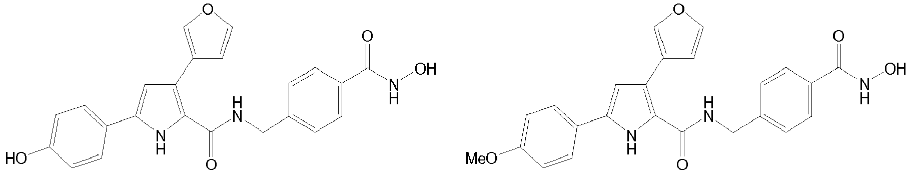 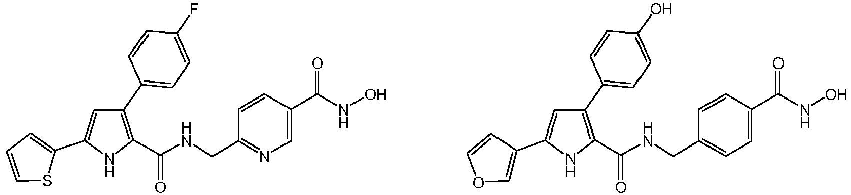 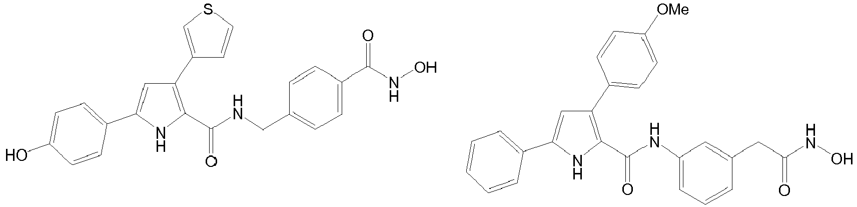 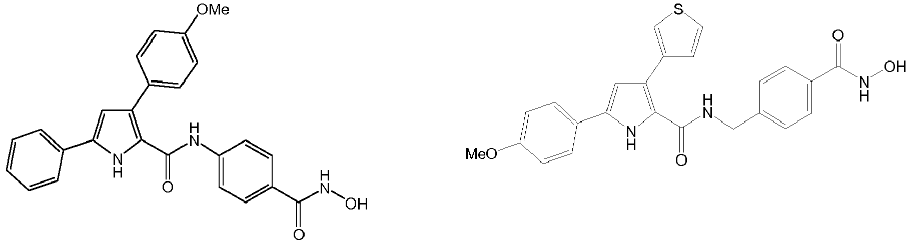 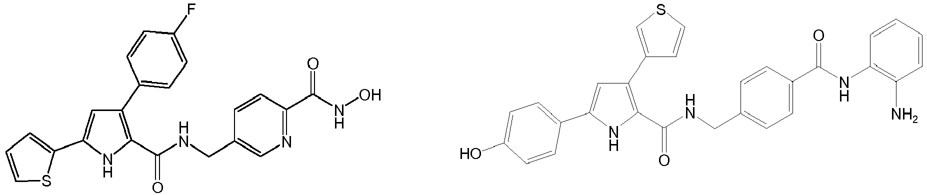 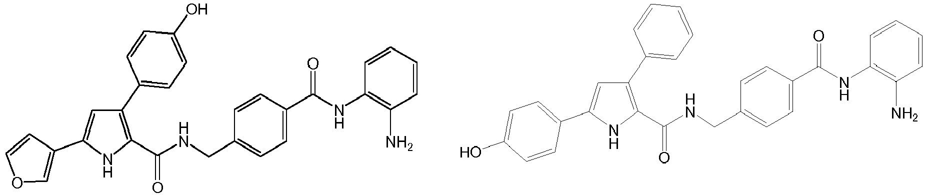 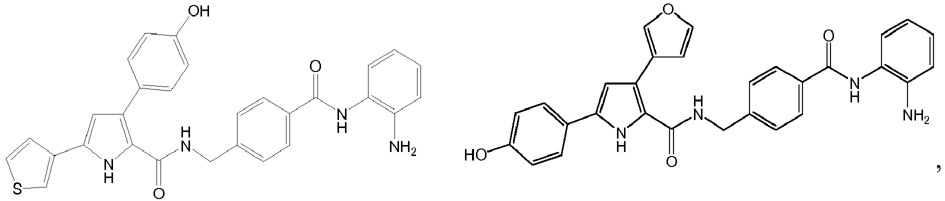 or a salt or solvate thereof.

6. A compound for use according to claim 1, selected from the following list: 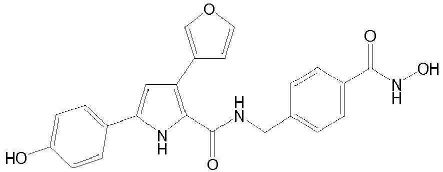 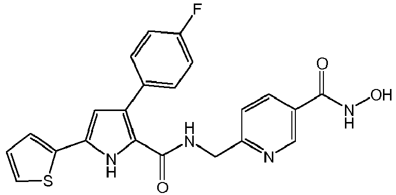 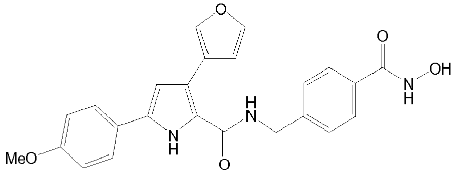 or a salt or solvate thereof.

7. A compound for use according to any preceding claim, which is 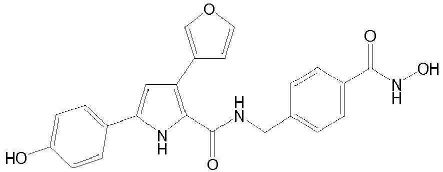 or a salt or solvate thereof.

8. A compound for use according to any preceding claim, which is

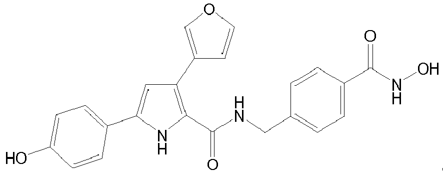

9. A compound for use according to any preceding claim, wherein the autoimmune disease is selected from autoimmune hepatitis; an inflammatory demyelinating disease of the central nervous system; systemic lupus erythematosus; acute anterior uveitis; Sjögren's syndrome; rheumatoid arthritis; diabetes mellitus type 1; Graves' disease; and inflammatory bowel disease.

10. A compound for use according to any preceding claim, wherein the autoimmune disease is selected from autoimmune hepatitis and an inflammatory demyelinating disease of the central nervous system.

11. A compound for use according to any preceding claim, wherein the autoimmune disease is an inflammatory demyelinating disease of the central nervous system.

12. A compound for use according to claims 9 to 11, wherein the inflammatory demyelinating disease of the central nervous system is selected from multiple sclerosis (MS); neuromyelitis optica; acute-disseminated encephalomyelitis; acute hemorrhagic leukoencephalitis; Balo concentric sclerosis; Schilder's disease; Marburg MS; tumefactive MS; solitary sclerosis; optic neuritis; transverse myelitis; Susac's syndrome; leukoaraiosis; myalgic encephalomyelitis; Guillain-Barré syndrome; progressive inflammatory neuropathy; and leukodystrophy.

13. A compound for use according to claim 12, wherein the inflammatory demyelinating disease of the central nervous system is multiple sclerosis or acute-disseminated encephalomyelitis.

14. A compound for use according to claim 13, wherein the disease is multiple sclerosis.

15. A compound for use according to claims 1-8, wherein the autoimmune disease is autoimmune hepatitis.
